Europäisches Patentamt

European Patent Office

Office européen des brevets

(19)

(11) **EP 1 224 486 B1**

(12) **EUROPEAN PATENT SPECIFICATION**

(45) Date of publication and mention
of the grant of the patent:
**28.12.2005 Bulletin 2005/52**

(51) Int Cl.[7]: **G01R 33/46**, G01N 24/08,
G01N 33/68, G01N 33/50

(21) Application number: **00972833.8**

(22) Date of filing: **19.10.2000**

(86) International application number:
**PCT/EP2000/010370**

(87) International publication number:
**WO 2001/033243 (10.05.2001 Gazette 2001/19)**

(54) **USE OF DOUBLE-QUANTUM 1H-NMR SPECTROSCOPY FOR THE IDENTIFICATION OF LIGANDS INTERACTING WITH TARGET BIOMOLECULES**

ANWENDUNG DER 1H-NMR-DOPPELQUANTENSPEKTROSKOPIE ZUR IDENTIFIZIERUNG VON LIGANDEN, DIE MIT ZIEL-BIOMOLEKÜLEN WECHSELWIRKEN

UTILISATION DE LA SPECTROSCOPIE QUANTIQUE DOUBLE 1H-RMN POUR L'IDENTIFICATION DE LIGANDS EN INTERACTION AVEC DES BIOMOLECULES CIBLES

(84) Designated Contracting States:
**AT BE CH CY DE DK ES FI FR GB GR IE IT LI LU MC NL PT SE**
Designated Extension States:
**AL LT LV MK RO SI**

(30) Priority: **29.10.1999 GB 9925677**

(43) Date of publication of application:
**24.07.2002 Bulletin 2002/30**

(73) Proprietor: **Pharmacia Italia S.p.A.**
**20152 Milano (IT)**

(72) Inventors:
• **DALVIT, Claudio**
**I-20154 Milano (IT)**
• **BATTISTINI, Carlo**
**I-20026 Novate Milanese (IT)**
• **CACCIA, Paolo**
**I-21047 Saronno (IT)**
• **GIORDANO, Patrizia**
**I-12100 Cuneo (IT)**
• **PEVARELLO, Paolo**
**I-27100 Pavia (IT)**
• **SUNDSTRÖM, Michael**
**11460 Stockholm (SE)**

• **TATO', Marco**
**I-20151 Milano (IT)**

(74) Representative: **Modiano, Micaela Nadia et al**
**Modiano, Josif, Pisanty & Staub Ltd.,**
**Baaderstrasse 3**
**80469 München (DE)**

(56) References cited:
**DE-C- 19 649 359          US-A- 5 891 643**

• **DALVIT C ET AL: "Heteronuclear X-Filter /sup 1/H PFG double-quantum experiments for the proton resonance assignment of a ligand bound to a protein" JOURNAL OF MAGNETIC RESONANCE, MARCH 1998, ACADEMIC PRESS, USA, vol. 131, no. 1, pages 148-153, XP002158775 ISSN: 1090-7807 cited in the application**
• **HAJDUK ET AL: "One-dimensional relaxation- and diffusion-edited NMR methods for screening compounds that bind to macromolecules" J. AM. CHEM. SOC., vol. 119, no. 50, 1997, pages 12257-12261, XP002158776 cited in the application**

**Description**

**Technical Field of the Invention**

The present invention relates to a method for the use of $^1$H two-dimensional double-quantum spectroscopy in the detection of chemical moieties that serve as scaffold of a potential drug that is directed to a target biomolecule.

**Background of the Invention**

The rapid identification of potential lead compounds is the first important step in the drug-discovery process. Often, lead molecules are identified by using biological screening techniques such as random or directed sublibrary high-throughput screening. The availability of proprietary chemical libraries and robotic systems for bioassay allows testing of hundred of thousands of compounds in few months.
However, these assays may suffer from artifacts originating from different sources of potential errors (e.g. detection system, cytotoxicity, not binding to the right target).
Over the last years NMR spectroscopy has emerged as a useful tool for identifying compounds interacting with the desired target[1-10] and some of these techniques are known in the art. See, for a reference, the international patent applications WO 97/18469, WO 97/18471 and WO 98/48264, all in the name of Abbott Laboratories, and WO 98/57155 in the name of Vertex Pharmaceuticals Inc.
These known methodologies can be essentially classified in two categories: those that monitor the signals of the protein[1.2.10] and those that monitor the signal of the ligands.[3-7] Both techniques, however, suffer from several shortcomings.
As an example, whilst the former typically makes use of $^{15}$N labelled protein targets and is currently limited only to proteins of molecular weight (<30 kDa), the latter uses one-dimensional (ID) NMR experiments for the identification of ligands among a mixture of molecules. In this respect, severe problems of overlapping in the 1D spectra of compound mixtures, particularly when containing more than ten compounds, make difficult the identification of molecules interacting with the target biomolecule.
A two-dimensional (2D) homonuclear method based on transfer NOE has been proposed for screening compound mixtures.[11,12] This technique has been disclosed in the German patent DE 19649359 (Peter Thomas). However, since the experiment is not sensitive and high ligand concentrations are required, major drawbacks arise when the ligand is not very soluble in water and/or when mixtures of many different compounds have to be tested, hence making difficult to reach the above ligand concentrations.
Dalvit, et al. *J. Magn. Reson.* **1998**, *131*, 148-153 discusses 2D double quantum experiments. However, the target/biomolecule requires $C^{13}$ and $N^{15}$ labeling.
Therefore, we propose a different NMR technique for a new, rapid, reliable and efficient means of screening compound mixtures in the identification of ligands that bind to a particular biomolecular target.

**Summary of the Invention**

The present invention provides a NMR procedure for screening compounds for their binding properties to a particular unlabelled target protein.
It is therefore the object of the present invention a method for providing a ligand which interacts with a target biomolecule, which method comprises:

   i) generating a two dimensional proton (refocussed or not) double-quantum or a DQC (double-quantum coherence) relaxation weighted double-quantum spectrum of a solution comprising one or more chemical molecules at least containing two doublets;
   ii) generating a two dimensional proton (refocussed or not) double-quantum or a DQC relaxation weighted double-quantum spectrum of the above solution further comprising the target biomolecule;
   iii) comparing spectrum in (i) with spectrum in (ii) so as to detect any differences that identify the presence of one or more chemical molecules that bind to the target biomolecule and selecting the said one or more chemical molecules as a ligand or ligands to the target biomolecule.

The identification of the molecular entity or entities interacting with the target biomolecule is obtained through the analysis of the two dimensional spectra as above defined in (i) and (ii).
According to a preferred embodiment of the present invention, an optional additional step involves the generation of two dimensional proton (refocussed or not) double-quantum spectra with and without the target protein for the compound, or for each of the compounds, which have been identified as ligands in the above step iii).

According to another preferred embodiment of the invention, the two dimensional proton (refocussed or not) double-quantum or DQC relaxation weighted double-quantum spectra are recorded with z or magic angle pulse field gradients.

The method exploits efficiently and simultaneously the different spin-spin and Double-Quantum coherence relaxations of the compounds that interact with a macromolecule. One of the avantages of the present invention is its capability to determine directly from the 2D spectra, hence without the need of deconvolution, those molecules that are binders to the target biomolecule. Another advantage resides in the good multiple solvent suppression obtained with the use of coherence selection gradients. Remarkable multiple solvent suppression is achieved with coherence selection pulsed field gradients tilted at the magic angle.

Therefore, the chemical mixtures can be prepared in non-deuterated solvents.

Other major advantages of the method of the invention are its high sensitivity and the fact that weak binders can also be identified.

In addition, this methodology can be advantageously applied to large target biomolecules with no limits in the upper size and does not require $^{15}N$ or $^{13}C$ labelled biomolecules.

As set forth above, an absolute requirement for this method is that the proton spectrum of the molecule, or molecules, to be screened contains at least two doublets. Nevertheless, since most of the molecules contain two protons that are scalar coupled with each other, this is certainly not a stringent requirement.

**Brief Description of the Drawings**

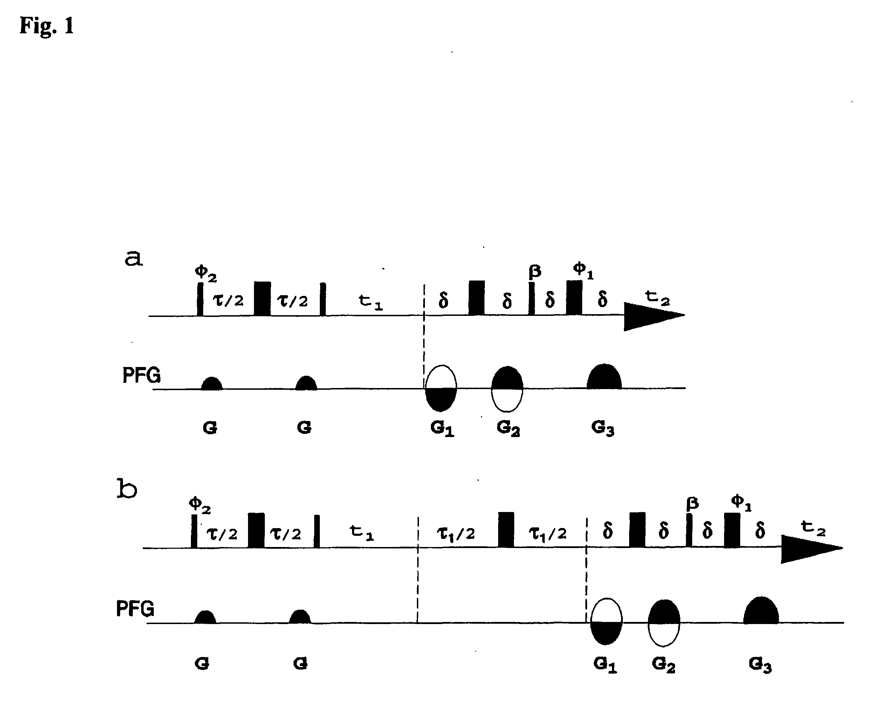

**Figure 1** depicts the 2D $^1H$ double-quantum (a) and DQC relaxation weighted double-quantum (b) pulse sequences (version with z or magic angle pulsed field gradients). The gradients $G_1$ to $G_3$ are the coherence selection gradients and they should be applied at the magic angle[13] if triple axis gradient hardware is available. The first two weak pulsed field gradients (PFG) are used for suppression of radiation damping. The delay $\tau$ is between 30 to 50 ms whereas the delay $\tau_1$ is set between 10 to 200 ms. A very weak (<20 Hz) $H_2O$ presaturation field can be applied before the excitation DQ period in the z-PFG version of the DQ experiments if the $H_2O$ suppression is not optimal. The technical details of the experiment together with the pulse sequence for the refocussed DQ experiment are found in a recent review article.[14]

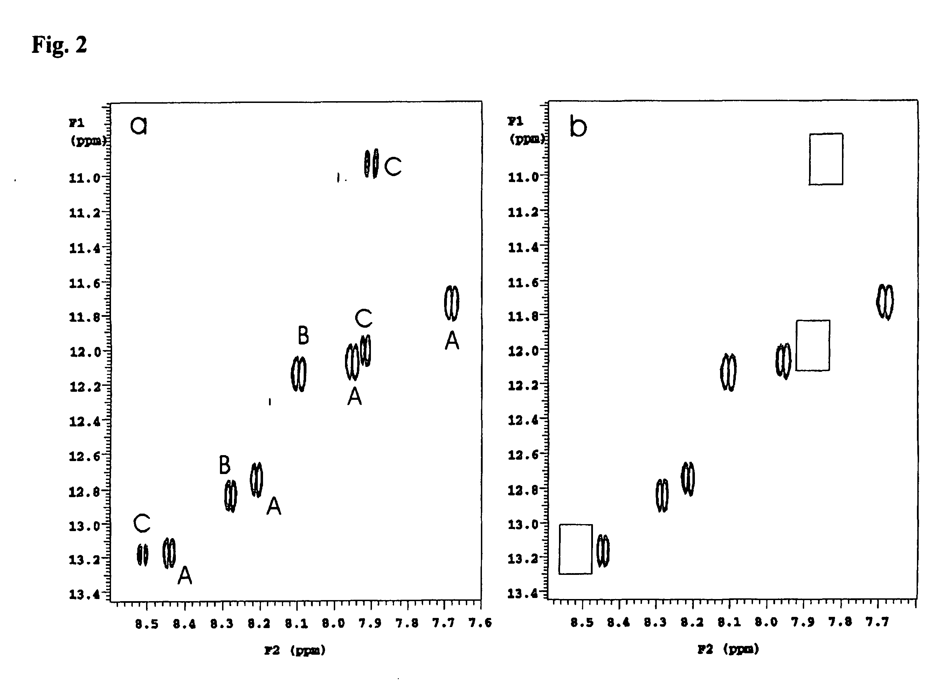

**Figure 2,** panel (a), depicts the fingerprint region of the 2D DQ spectrum of mixture 1 (Ac-Tyr-Val-Asn-Val-OH (resonances A), Ac-Tyr($PO_3H_2$)-Val-$NH_2$ (resonances B) and Ac-(N-Me)Phe(p-$CH_2PO_3H_2$)-Val-Asn-NH-$CH_2$-CH($CH_3$)$_2$ (resonances C)) in the absence of the target protein. Figure 2, panel (b), depicts the same 2D DQ fingerprint region of the mixture in the presence of the Grb2 SH2 domain. The delay $\tau$ was 42 ms long. Each of the molecules is present at a molar ratio to the target protein of 2:1 (100 μM : 50 μM). The rectangles in (b) indicate the position of the missing cross peaks.

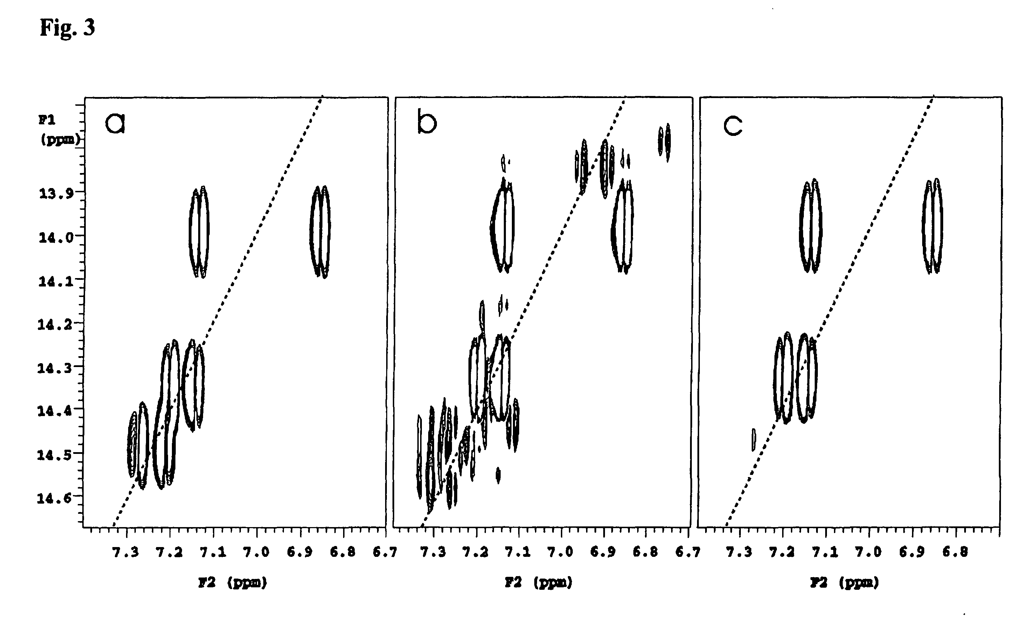

**Figure 3,** panel (a), depicts the aromatic region of the 2D DQC relaxation weighted double-quantum spectrum of mixture 1 (Ac-Tyr-Val-Asn-Val-OH (resonances A), Ac-Tyr($PO_3H_2$)-Val-$NH_2$ (resonances B) and Ac-(N-Me)Phe(p-CH2PO3H2)-Val-Asn-NH-CH2-CH(CH3)2 (resonances C)) in the absence of the target protein. Figure 3, panel (b) and (c) depicts the same spectral region of the 2D double-quantum and DQC relaxation weighted double-quantum spectrum of mixture 1 in the presence of the Grb2 SH2 domain, respectively. The delay $\tau$ and $\tau_1$ were 42 and 24 ms long, respectively The dashed line corresponds to the double-quantum pseudodiagonal. The direct peaks are disposed symmetrically with respect to the diagonal. The missing ligand resonances in (b) and (c) are those of the aromatic moiety of compound C.

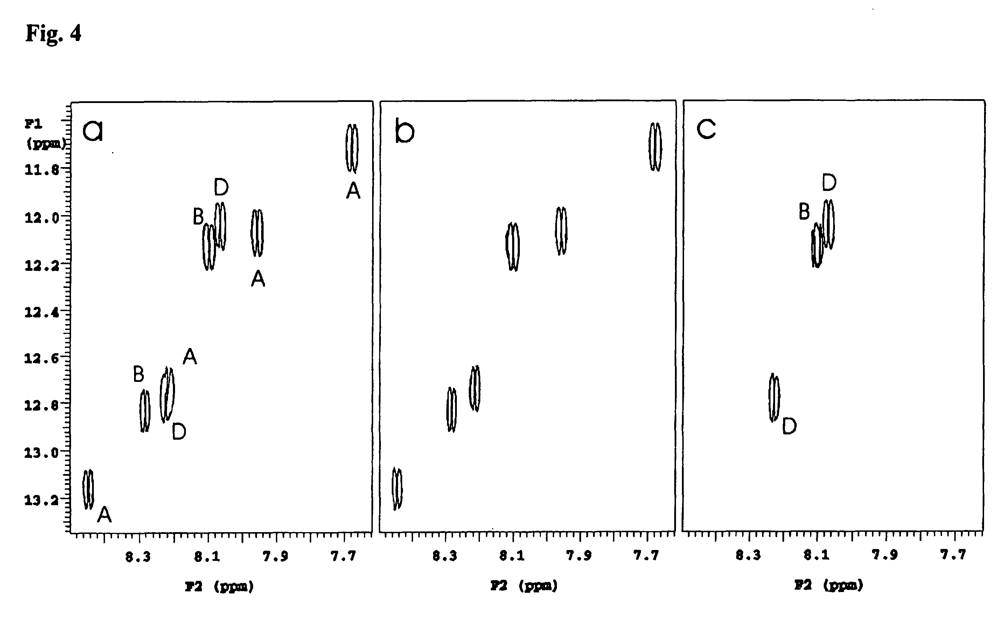

**Figure 4,** panel (a), depicts the fingerprint region of the 2D DQ spectrum of mixture 2 (Ac-Tyr-Val-Asn-Val-OH (resonances A), Ac-Tyr($PO_3H_2$)-Val-$NH_2$ (resonances B), indole (resonances C) and Ac-Tyr($PO_3H_2$)-Val-NH-$CH_2$-$CH_2$-$CONH_2$ (resonances D)). The resonances of indole are not visible in this spectral region. Figure 4, panel (b), depicts the same 2D DQ fingerprint region of the four compound mixture in the presence of the Grb2-SH2 domain. Figure 4, panel (c), depicts the 2D difference spectrum obtained by subtracting the two 2D DQ spectra (a) and (b). The visible signals in (c) are those of compound C that disappear in the spectrum (b) and one signal of compound B that displays a small chemical shift change in the presence of the protein. This is in indication of a possible weak interaction between compound B and the Grb2-SH2 domain. Each of the molecules is present at a molar ratio to the target protein of 6:1 (300 μM : 50 μM). The resonances of the indole are not affected by the presence of the protein (data not shown).

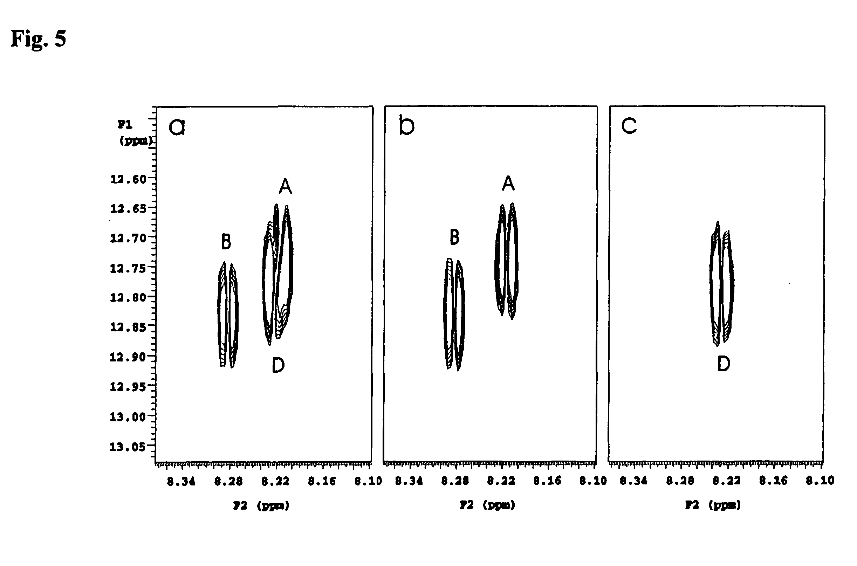

**Figure 5** depicts an expanded region of the 2D DQ spectrum of mixture 2 in the absence (a) and in the presence of the protein Grb2-SH2 (b). The spectrum in (c) depicts the 2D difference spectrum obtained by subtracting the two 2D DQ spectra (a) and (b). Other experimental parameters are same as described in Figure 4. Despite the strong overlap of cross peaks A and D in (a) it is possible to identify in the 2D DQ difference spectrum (c) the cross peak that is affected by the presence of the protein.

### Detailed Description of the Invention

The present invention provides a rapid and efficient screening procedure for identifying and providing chemical moieties that interact with a target biomolecule, for instance a therapeutic target biomolecule.
The molecules are identified by following with 2D $^1$H DQ or DQC (refocussed or not) relaxation weighted DQ experiments (preferentially version with PFG) the changes in either the intensities or chemical shifts of the ligand signals in the presence of the target biomolecule.
The target biomolecule is either free in solution, dissolved in detergents or micelles or, alternatively, coupled to a resin. For the resin samples, two-dimensional Magic Angle Spinning (MAS) DQ or DQC relaxation weighted DQ experiments (preferentially version with PFG) are recorded.
The target biomolecule according to the method object of the present invention is any biomolecule which is sufficiently larger in size in comparison to the ligand and capable of binding to the selected ligand.
Suitable biomolecules are, for instance, proteins, peptides, antibodies, DNA or RNA fragments and the like.
Preferably, the target biomolecule is a protein.
For ligands in fast exchange on the NMR time scale (with $K_D$ >30-50 µM), a change in chemical shift ($\Delta\Omega$) is observed in the presence of the target biomolecule, e.g. the protein. The $\Delta\Omega$ is the population-weighted average of the chemical shifts for the free and bound states of the chemical moiety. Ligands with higher affinities for the target biomolecules can exhibit intermediate exchange ($K_D$ in the few µM range) or slow exchange ($K_D$ < 1 µM). In these cases the ligand resonances will be attenuated or broadened in the 2D DQ spectra recorded in the presence of the protein. The pulse sequences for the 2D $^1$H DQ and DQC relaxation weighted DQ experiments (versions with PFG) are shown in Fig. 1a and 1b, respectively.
Let us consider two spins 1 and 2 of a ligand that interacts with a target macromolecule. The two spins are scalar coupled with a $J_{12}$ coupling constant. The detected magnetization of spin 1 $M_1^+$ $(t_1,t_2)$ created by magnetization of spin 1 $M_1^+$ (0) with the pulse sequence of Fig. 1a and Fig. 1b is given respectively by:

$$M_1^+(t_1,t_2) \propto M_1^+(0)f(J_{12}.\tau.\beta)\exp(-\frac{\tau}{T_2^{SQ}})\exp(-\frac{t_1+2\delta}{T^{DQ}})\exp(\frac{t_2+2\delta}{T_2^{SQ}})\exp(i\Omega_{DQ}t_1)\exp(i\Omega_{SQ}t_2) \tag{1}$$

$$M_1^+(t_1, t_2) \propto M_1^+(0)f(J_{12}, \tau, \beta)\exp(-\frac{\tau}{T_2^{SQ}})\exp(-\frac{t_1+\tau_1+2\delta}{T^{DQ}})\exp(-\frac{t_2+2\delta}{T_2^{SQ}})\exp(i\Omega_{DQ}t_1)\exp(i\Omega_{SQ}t_2) \tag{2}$$

Similar equations are derived for detected magnetization of spin 1 $M^+_1$ $(t_1, t_2)$ created by magnetization of spin 2 $M^+_2(0)$. In deriving equations (1) and (2) we have assumed that the parallel transitions of the multiplets have the same spin-spin relaxation. In other words we have considered the $^1$H DD-CSA cross correlation term as negligible.
f is a trigonometric function that represents the efficiency of the forward and backward conversion between single quantum (SQ) and double quantum coherence. For a two-spin system the trigonometric function for the echo pathway selection is [14,18-20]:

$$f(J_{12},\tau,\beta) = \frac{1}{2}\sin \pi J_{12}\tau \sin \beta(\cos \beta-1) \tag{3}$$

and for the anti-echo pathway selection is:

$$f(J_{12}, \tau, \beta) = \frac{1}{2}\sin \pi J_{12}\tau \sin \beta(\cos \beta + 1) \tag{4}$$

where $\beta$ is the angle of the observation pulse and $\tau$ is the excitation DQ period. $\Omega_{DQ}$ and $\Omega_{SQ}$ are the chemical shifts of DQ and SQ coherence, respectively. $T_2^{SQ}$ and $T^{DQ}$ are the experimentally observed spin-spin relaxation of spin 1 and the Double-Quantum relaxation, respectively. The other terms in equations (1) and (2) are described in Figure 1. The observed $T_2^{SQ}$ and $T^{DQ}$ are the average of the proton relaxations of the free molecule and when complexed with the protein weighted by the mole fractions of the two populations. One and three additional exchange terms contribute in situation of intermediate exchange to the observed relaxation of (SQ) and (DQ) coherence, respectively.[16] Therefore the binding of a small molecule to a target macromolecule will result in shorter $T_2^{SQ}$ and $T^{DQ}$ (i.e. the signals of the cross peaks will broaden in both dimensions). The broadening in $\omega_1$ is due to a shortening of $T^{DQ}$ (for large molecules the DQ relaxation is very rapid[21]) and in $\omega_2$ is due to a shortening of $T_2^{SQ}$. This broadening will be measured by comparing 2D DQ spectra of the ligand with and without the protein present.

From a practical point of view, the steps involved in the detection of binding are preferably the following:

a) mixing together from 1 to n chemical compounds not interacting with each other;

b) recording a two-dimensional $^1$H DQ or DQC (refocussed or not) relaxation weighted DQ spectrum (preferentially version with PFG) for the said compound mixture in the absence of the target biomolecule;

c) mixing the said compound mixture with the target biomolecule where each of the molecules comprising the mixture is preferably present at a molar ratio to the target biomolecule of about 1:1 to about 20:1

d) recording a two-dimensional $^1$H DQ or DQC (refocussed or not) relaxation weighted DQ spectrum (preferentially version with PFG) for the said compound mixture in the presence of the target biomolecule;

e) comparing the spectra recorded in steps b) and d) to detect which, if any of the chemical entities of the mixture, has interacted with the target biomolecule; and, optionally

f) recording a two-dimensional $^1$H DQ or DQC (refocussed or not) relaxation weighted DQ spectrum (preferentially version with PFG) for each of the compounds which have been detected in step (e) above, with and without the target protein.

The optional additional step (f) which involves the generation of a two dimensional proton double-quantum spectra with and without the target protein for the compound, or for each of the compounds, identified as ligand(s), might be useful in rather complex mixtures so as to confirm the finding of step (e), that is the binding between the ligand and the target biomolecule.

From the foregoing, since the method of the present invention can be applied as well to one or more, that is from 1 to n chemical moieties which could interact to the target biomolecule, it is clear to the man skilled in the art that step (f) would be particularly useful with n>1 compounds to be tested.

The method of the invention is applied to any mixture of compounds to be tested wherein said mixture preferably comprises from 1 to 10 or, even more preferably, from 1 to 5 compounds.

The present invention based on 2D DQ spectroscopy for the screening has several unique advantages which are shortly listed below.

- The experiment, in particular the PFG versions recorded with $\beta=45°/135°$ or $\beta=60°/120°$, represents the most sensitive 2D NMR experiment for analysis of small and medium size molecules.

- A concentration in the order of 50 to 100 $\mu$M is sufficient for obtaining good quality 2D DQ spectra in relatively short time. The utilization of the cryoprobe technology[22-24] with 2D PFG DQ spectroscopy allows one to reduce currently the necessary ligand concentration to about 13 to 25 $\mu$M. Therefore, this permits the preparation of complicated mixtures comprising many molecules and avoids changes in the pH of the solution, e.g. of the aqueous buffered solution, for instance originating from the presence of organic acids or bases. The low ligand concentration needed for these experiments permits the use of hydrophobic compounds that otherwise at higher concentrations would be insoluble in aqueous solutions. Most important, the high sensitivity of the experiment coupled with the cryoprobe technology reduces the amount of precious target biomolecule, e.g. protein, needed for the screening. The protein concentration for each sample can be as low as from about 1 to about 5 $\mu$M.

- The method exploits the combined use of the two physical properties $T_2^{SQ}$ and $T^{DQ}$, as shown in equations (1) and (2), and therefore it represents a sensitive tool for detection of weak ligands. The difference in $T^{DQ}$ for a molecule free in solution and when complexed with a macromolecule is very large.

- Good solvent suppression is obtained with the use of coherence selection gradients. Remarkable multiple solvent suppression is then achieved with coherence selection gradients tilted at the magic angle. This becomes particularly relevant in the observation of very diluted samples, as in screening by NMR where the dynamic range of the receiver needs to be maximized in an optimal way. Furthermore, the excellent multiple solvent suppression allows the preparation of the chemical mixtures stock solutions in non-deuterated solvents (i.e. DMSO, $CH_3OH$). Another important advantage of the use of samples dissolved in $H_2O$ is the observation of the NH doublet exchangeable resonances. These signals are absent in the spectra recorded in $D_2O$ solutions. The NH proton signals are in a spectral region with no or little overlap with other signals and therefore can be used for assignment purposes in complicated mixtures. Furthermore the difference in chemical shift of a ligand NH free in solution and when hydrogen bonded to a protein is typically large whereas this difference can be very small for other protons of the same ligand. The large chemical shift difference helps in identifying weak ligands via the NH resonances either due to a more pronounced broadening deriving from a larger chemical exchange contribution to the observed $T_2^{SQ}$ and $T^{DQ}$ relaxation or due to a detectable chemical shift change. The latter is probably the explanation for the identification in Fig. 4 of compound Ac-Tyr(PO$_3$H$_2$)-Val-NH$_2$ as a very weak ligand.

- The 2D DQ experiment is a cosine modulated experiment and therefore maximum signal is observed in the first $t_1$ increments. The experiment can be recorded rapidly by acquiring only a limited number of $t_1$ increments. Linear prediction can then be applied in the $t_1$ dimension for improving the resolution.
  Extensive folding can also be performed in $\omega_1$ therefore reducing the spectral width and the number ot $t_1$ increments.
- Removal of the extensive overlapping present in the one-dimensional spectra allows an easy identification, in the 2D DQ spectrum, of the signals affected by the presence of the target biomolecule. Due to the reduced problem of overlapping and to the complete absence of biomolecule signals, the 2D DQ difference spectrum is less subject to artefacts when compared to the 1D difference spectrum.
  Furthermore, the absence of strong diagonal peaks and singlet resonances with consequent absence of $t_1$ noise and the excellent solvent suppression in DQ spectra allow a more precise and reliable use of the 2D subtraction technique.
- The scalar connectivities observed in the 2D DQ spectrum allow direct identification of the molecules interacting with the target biomolecule without the need to deconvolute the mixture.
- Suppression of the biomolecule signals is very efficient not only due to the presence of the excitation double quantum period and the relaxation of DQC during $t_1$ and $\tau_1$, but also due to the antiphase character of the multiplets along $\omega_2$. The large linewidth of the protein resonances results in extensive cancellation of the positive and negative lobes along $\omega_2$.

The NMR method of the invention, based on the newly optimized $^1$H Double-Quantum spectroscopy, for the identification of compounds that interact with a target biomolecule was described. The invention exploits efficiently and simultaneously the different spin-spin and Double-Quantum coherence relaxations of the compounds that interact with a macromolecule.
The high sensitivity, remarkable multiple solvent suppression, absence of diagonal peaks and singlets, reduced problems of resonance overlapping, all allow reliable application of this methodology to the screening of complex chemical mixtures against a target biomolecules. Both strong and weak ligands can be directly identified from a mixture.
The following examples are intended to better illustrate the present invention without pose any limitation to it.

**Examples**

The feasibility of the invention in identifying ligands was tested with mixtures of compounds against the Grb2-SH2 domain. This represents a challenging test case due to the small size of the protein employed (MW=11839). It should be noticed that the technique performs even better with medium or large size target proteins due to the very short $T_2^{SQ}$ and $T^{DQ}$ for these proteins.
Figure 2 illustrates the DQ fingerprint region of a compound mixture (mixture 1) recorded with the pulse sequence of Figure 1a in the absence (a) and in the presence (b) of the protein. Mixture 1 contained a final concentration of 100 µM of Ac-Tyr-Val-Asn-Val-OH, Ac-Tyr(PO$_3$H$_2$)-Val-NH$_2$ and Ac-(N-Me)Phe(p-CH$_2$PO$_3$H$_2$)-Val-Asn-NH-CH$_2$-CH(CH$_3$)$_2$-
Close comparison of the two spectra allows rapid identification of compound Ac-(N-Me)Phe(p-CH$_2$PO$_3$H$_2$)-Val-Asn-NH-CH$_2$-CH(CH$_3$)$_2$ (resonances C) as a ligand for Grb2-SH2. The NH protein resonances are not visible in Figure 2 (b) due to their short $T_2^{SQ}$ and $T^{DQ}$ and the lower protein concentration (50 µM).
The protein aromatic resonances have a longer $T_2^{SQ}$ and $T^{DQ}$ compared to the amide resonances and their presence in the DQ spectrum may interfere with the observation of the ligand signals. It is possible to suppress them by employing a DQC relaxation filter as shown in Figure 1b.
Figure 3(a) illustrates the aromatic region of the 2D relaxation weighted DQ spectrum for mixture 1. Figures 3 (b) and (c) depict the same spectral region of the 2D DQ and relaxation weighted DQ spectrum of mixture 1 in the presence of the Grb2 SH2 domain, respectively. The residual aromatic protein signals in (b) are completely suppressed in (c) due to the rapid DQ relaxation of the protein resonances. The missing ligand signals in (b) and (c) are those of the aromatic moiety of compound Ac-(N-Me)Phe(p-CH$_2$PO$_3$H$_2$)-Val-Asn-NH-CH$_2$-CH(CH$_3$)$_2$.
It should be pointed out that for medium and large size proteins for membrane bound proteins or for proteins bound to a resin the DQC relaxation filter is not necessary. The large linewidth of all the protein resonances and the antiphase character of the DQ multiplets along $\omega_2$ results in complete cancellation of the positive and negative lobes. Nevertheless the DQC relaxation filter can be used also in the detection of very weak ligands by exploiting the large differences in $T^{DQ}$ for molecules interacting with a target macromolecule.
Figure 4 demonstrates the power of 2D DQ difference spectroscopy in the identification of very weak ligands. The DQ fingerprint region of Mixture 2 (300 µM of Ac-Tyr-Val-Asn-Val-OH, Ac-Tyr(PO$_3$H$_2$)-Val-NH$_2$, Ac-Tyr(PO$_3$H$_2$)-Val-NH-CH$_2$-CH$_2$-CONH$_2$ and indole) in the absence (a) and in the presence of the protein (b) recorded with the pulse sequence of Figure la is shown. Although visual comparison allows immediate identification of compound Ac-Tyr $(PO_3H_2)$-Val-NH-$CH_2$-$CH_2$-$CONH_2$ (resonances D) as a potent ligand, the 2D difference spectrum (c) permits also the identification of compound Ac-Tyr$(PO_3H_2)$-Val-$NH_2$ as a potential very weak ligand. The small chemical shift change observed for the NH of Val is about 8Hz that is equal to the value of $^3J_{NH\alpha H}$. Therefore the signal in the difference spectrum appears as a triplet of intensity -1,+2,-1. Only the compound resonances that display changes in intensity or chemical shift in the presence of the protein will be visible in the difference spectrum. The resonances of compound Ac-Tyr-Val-Asn-Val-OH (resonances A) and Indole (not present in this spectral region) that do not interact with the protein are not visible in the difference spectrum.

The absence of diagonal peaks, $t_1$ noise and singlets and the excellent solvent suppression make the 2D DQ difference spectroscopy a very reliable technique. This can be appreciated in Figure 5 that shows an expanded region of the DQ spectra recorded for mixture 2. Despite the strong overlap of cross peaks A and D in (a) it is possible to identify peak D in the DQ difference spectrum (c) as the cross peak that is affected by the presence of the protein.

All the spectra were acquired at 25°C with the pulse sequences of Figure 1 using a Varian Inova 600 MHz spectrometer equipped with a 5 mm triple-resonance inverse probe and actively shielded x, y, and z- gradient coils. The DQ spectra were collected using 8 to 32 transients for each of the 256 $t_1$ increments (128 $t_1$ values for both the echo and antiecho pathway selections) and with 2048 points in $t_2$. The data were multiplied with a cosine window function in both dimensions prior to the Fourier transformation.

The excitation DQ period $\tau$ was 42 ms long and the angle $\beta$ of the detection pulse was set to 45° and 135° for the antiecho and echo coherence pathway selection, respectively. The coherence selection gradients $G_1$ to $G_3$ were set at the magic angle using only the x and z components of the gradients. The first two PFGs applied at the beginning and end of the excitation double-quantum period were weak gradients for radiation damping suppression. The 2D spectral differencing was performed with the software provided by Varian. The scaling factor used in the examples reported here was -1. However it can be adjusted manually for allowing small variations in concentration of the protein-ligand mixture and ligand mixture solutions.

Two different compound mixtures consisting of three and four molecules were prepared. Mixture 1 contained a final concentration of 100 µM of Ac-Tyr-Val-Asn-Val-OH, Ac-Tyr$(PO_3H_2)$-Val-$NH_2$ and Ac-(N-Me)Phe(p-$CH_2PO_3H_2$)-Val-Asn-NH-$CH_2$-$CH(CH_3)_2$ while mixture 2 contained 300 µM of Ac-Tyr-Val-Asn-Val-OH, Ac-Tyr$(PO_3H_2)$-Val-$NH_2$, Ac-Tyr $(PO_3H_2)$-Val-NH-$CH_2$-$CH_2$-$CONH_2$ and indole. Some of these small peptidomimetics have been shown to bind to the SH2 domain of Grb2.[25-27]

The peptides and related peptidomimetics have been synthesized by solid phase methodology. The protected peptide on resin was synthesized by sequential coupling of the N-Fmoc protected amino acids, some of them also protected at the side chain, starting from the C-terminal. After the last coupling the tyrosine residue was phosphorylated on solid phase by using phosphoramidite chemistry and successive oxidation. The phosphorylation was followed by acidic treatment leading to side-chain deprotection and detachment from the resin.

The SH2 domain of Grb2 was expressed in *E. coli* as GST fusion proteins with a recognition site for cleavage with rhinovirus Prescission Protease (Amersham Pharmacia Biotech). The protein was present in the supernatant of the cell lysate after bacterial cell disruption and centrifugation. The supernatant containing the fusion protein was loaded onto a Glutathione Sepharose resin and bacterial contaminants eliminated after washing with PBS. To remove the GST moiety the resin carrying the GST-SH2 was incubated with Prescission Protease. The SH2 domain was further purified to homogeneity using Superdex 75 gel filtration chromatography. The protein concentration for the NMR experiments was 50 µM. The samples were in aqueous (95% $H_2O$ / 5% $D_2O$) buffered solutions (50 mM Phosphate, 100 mM NaCl pH 6.2 and 0.02% $NaN_3$.).

**References:**

(1) Shuker, S.B.; Hajduk, P.J.; Meadows R.P.; Fesik, S.W. *Science,* **1996,** *274,* 1531-1534.

(2) Hajduk, P. J.; Sheppard, D. G.; Nettesheim, D. G.; Olejniczak, E. T.; Shuker, S. B.; Meadows, R. P.; Steinman, D. H.; Carrera, G. M., Jr.; Marcotte, P. M.; Severin, J.; Walter, K.; Smith, H.; Gubbins, E.; Simmer, R.; Holzman, T. F.; Morgan, D. W.; Davidsen, S. K.; Summers, J. B.; Fesik, S. W. *J. Am. Chem. Soc.* **1997,** 119, 5818-5827.

(3) Hajduk, P.J.; Olejniczak E.T.; Fesik, S.W. *J. Am. Chem. Soc.,* **1997,** *119,* 12257-12261.

(4) Lin, M.; Shapiro, M.J.; Wareing, J.R. *J. Am. Chem. Soc.* **1997,** 119, 5249-5250.

(5) Dalvit, C.; Ramage, P.; Hommel, U. *J. Magn. Reson.* **1998***, 131,* 148-153.

(6) Bleicher, K.; Lin, M.; Shapiro, M. J.; Wareing, J. R. *J. Org. Chem.* **1998***, 63, 8486*-8490.

(7) Gonnella, N.; Lin, M.; Shapiro, M. J.; Wareing, J. R.; Zhang, X. *J. Magn. Reson.* **1998,** *131,* 336-338.

(8) Stockman, B. J. *Progress in Nuclear Magnetic resonance Spectroscopy,* **1998,** 33, 109-151.

(9) Moore, J.M. *Analytical Biotechnology,* **1999,** 10, 54-58.

EP 1 224 486 B1

(10) Hajduk, P. J.; Gerfin, T.; Bohlen, J.-M.; Haeberli, M.; Marek, D.; Fesik, S. W. *J. Med. Chem.*, **1999,** *42,* 2315-2317.

(11) Meyer, B.; Weimar, T.; Peters, T. *Eur. J. Biochem.* **1997,** 246, 705-709.

(12) Henrichsen, D.; Ernst, B.; Magnani, J. L.; Wang, W.-T.; Meyer, B.; Peters, T.; *Angew. Chem. Int. Ed,* **1999**, 38,98-102.

(13) Dalvit, C.; Böhlen, J. M. *J. Magn. Reson. B* **1996,** 111, 76-80.

(14) Dalvit, C.; Böhlen, J. M. in *Annual Reports on NMR Spectroscopy,* (Webb, A.; Ed.), Academic Press, **1999,** Vol. 37, pp 204-271.

(15) Feeney, J.; Batchelor, G.; Albrand, J. P.; Roberts G. C. K. *J. Magn. Reson.* **1979***,* 33, 519-529.

(16) Lian L. Y.; Roberts G. C. K. in *NMR of Macromolecules* (G.C.K. Roberts, Ed.) Oxford University Press, **1993,** pp. 153-182.

(17) Feeney J.; Birdsall B. in *NMR of Macromolecules* (G.C.K. Roberts, Ed.) Oxford University Press **1993**, pp. 183-215.

(18) Braunschweiler, L.; Bodenhausen, G.; Ernst, R. R. *Mol. Phys.,* **1983***, 48,* 535-560.

(19) Mareci, T. H.; Freeman, R. *J. Magn. Reson.,* **1983**, *51,*531-535.

(20) Ernst, R. R.; Bodenhausen, G.; Wokaun, A. *Principles of Nuclear Magnetic Resonance in One and Two Dimensions,* Clarendon Press, Oxford, 1987.

(21) Konrat, R.; Sterk, H. *Chem Phys. Lett.,* **1993,** 203, 75-80.

(22) Styles, P.; Soffe, N. F.; Scott, C. A.; Cragg, D. A.; Row, F.; White, D. J.; White, P.C.J. *J. Magn. Res.* **1984***, 60,* 397-404*.*

(23) Black, R.D.; Early, T.A.; Roemer, P.B.; Mueller, O.M.; Mogro-Campero, A.; Turner, L.G.; Johnson, G.A. *Science* **1993,** 259, 793.

(24) Marek, D.; Haeberli, M.; Triebe, R.; Baselgia, L.; Gerfin, T.; Schett, O.; Laukien, F. *ENC97* **1997;** PosterP122.

(25) Battistini, C.; Giordano, P.; De Rosa, S.; Corradi, F.; Comoglio, P.; Bardelli, A.; Intern. Appl. WO 97/30079 (Aug. 21, 1997). Priority GB application UK 9603227.1

(26) Schiering, N.; Casale, E.; Giordano, P.; De Rosa, S.; Caccia, P.; Sgarella, L.; Bolis, G.; Battistini, C. Poster at Keystone Symposium: Temporal and Spatial Determinants of specificity in Signal Transduction, Keystone (CO) USA, March 31 - April 16, 1997.

(27) Battistini C.; Giordano, P.; De Rosa, S.; Corradi, F.; Bardelli, A.; Gramaglia, D.; Comoglio, P. Poster at Keystone Symposium: Temporal and Spatial Determinants of specificity in Signal Transduction, Keystone (CO) USA, March 31 - April 6, 1997.

## Claims

1. A method for screening compounds for their binding properties to a target biomolecule, which method comprises:

   i) generating a two dimensional proton double-quantum or a DQC (double-quantum coherence) relaxation weighted double-quantum spectrum of a solution comprising one or more chemical molecules at least containing two doublets ;
   ii) generating a two dimensional proton double-quantum or a DQC relaxation weighted double-quantum spectrum of the above solution further comprising the target biomolecule;
   iii) comparing spectrum in (i) with spectrum in (ii) so as to detect any differences that identify the presence of one or more chemical molecules that bind to the target biomolecule and wherein the two dimensional proton double-quantum spectrum is refocused or not.

2. A method according to claim 1 comprising the additional step of generating two dimensional proton double-quantum spectra with and without the target protein for the compound, or for each of the compounds, which have been identified as ligands, to the target biomolecule.

3. A method according to claim 2 wherein the differences that identify the presence of one or more chemical molecules that bind to the target biomolecule is in the chemical shift or intensity of one or more signals.

4. A method according to any one of the preceding claims wherein each of the two dimensional proton double-quantum or DQC relaxation weighted double-quantum spectra are recorded with z or magic angle pulsed field gradients.

5. A method according to any one of the preceding claims wherein the two dimensional proton double-quantum or DQC relaxation weighted double-quantum spectra are recorded in non-deuterated solvents.

6. A method according to claim 1 wherein each test compound is present in the sample in a molar ratio to the target biomolecule of from about 1:1 to about 20:1.

7. A method according to claim 1 wherein the target biomolecule is free in solution, dissolved in detergents or micelles, or coupled to a resin.

8. A method according to claim 1 wherein the target biomolecule is a protein, an antibody or a DNA or RNA fragment.

9. A method according to claim 8 wherein the target biomolecule is a protein.

10. A method according to claim 9 wherein the protein is the SH2 domain of Grb2 protein.

**Patentansprüche**

1. Ein Verfahren zum Screening von Verbindungen nach ihren Bindungseigenschaften an ein Ziel-Biomolekül, wobei das Verfahren folgendes umfasst:

   i) Erzeugen eines zweidimensionalen Proton-Doppelquanten- oder eines DQC (Doppelquanten-Kohärenz) -Relaxations-gewichteten Doppelquanten-Spektrums von einer Lösung, die eines oder mehrere chemische Moleküle umfasst, die mindestens zwei Dubletten enthalten;
   ii) Erzeugen eines zweidimensionalen Proton-Doppelquanten- oder eines DQC-Relaxations-gewichteten-Doppelquanten-Spektrums der obigen Lösung, die weiterhin das Ziel-Biomolekül umfasst;
   iii) Vergleichen des Spektrums in (i) mit dem Spektrum in (ii), um irgendwelche Unterschiede zu detektieren, die die Anwesenheit von einem oder mehreren chemischen Molekülen nachweisen, die an das Ziel-Biomolekül binden, und worin das zweidimensionale Proton-Doppelquanten-Spektrum refokussiert ist oder nicht.

2. Ein Verfahren gemäß Anspruch 1, das den zusätzlichen Schritt Erzeugen von zweidimensionalen Proton-Doppelquanten-Spektren mit und ohne dem Ziel-Protein für die Verbindung, oder für jede der Verbindungen, welche als Liganden für das Ziel-Biomolekül identifiziert wurden, umfasst.

3. Ein Verfahren gemäß Anspruch 2, worin die Unterschiede, die die Anwesenheit von einem oder mehreren chemischen Molekülen, die an das Ziel-Biomolekül binden, nachweisen, in dem chemischen Shift oder der Intensität von einem oder mehreren Signalen liegen.

4. Ein Verfahren gemäß irgendeinem der vorhergehenden Ansprüche, worin jedes der zweidimensionalen Proton-Doppelquanten- oder DQC-Relaxations-gewichteten-Doppelquanten-Spektren mit z oder magischer Winkel gepulsten Feld-Gradienten aufgezeichnet wird.

5. Ein Verfahren gemäß irgendeinem der vorhergehenden Ansprüche, worin die zweidimensionalen Proton-Doppelquanten- oder DQC-Relaxations-gewichteten-Doppelquanten-Spektren in nicht-deuterierten Lösungsmitteln aufgezeichnet werden.

6. Ein Verfahren gemäß Anspruch 1, worin jede Testverbindung in der Probe in einem molaren Verhältnis zum Ziel-Biomolekül von ungefähr 1:1 bis ungefähr 20:1 vorhanden ist.

7. Ein Verfahren gemäß Anspruch 1, worin das Ziel-Biomolekül frei in Lösung vorliegt, aufgelöst in Reinigungsmitteln oder Mizellen, oder an ein Harz gekoppelt ist.

8. Ein Verfahren gemäß Anspruch 1, worin das Ziel-Biomolekül ein Protein, ein Antikörper oder ein DNA- oder RNA-Fragment ist.

9. Ein Verfahren gemäß Anspruch 8, worin das Ziel-Biomolekül ein Protein ist.

10. Ein Verfahren gemäß Anspruch 9, worin das Protein die SH2-Domain von Grb2-Protein ist.

**Revendications**

1.  Procédé pour sélectionner des composés en ce qui concerne leurs propriétés de liaison à une biomolécule cible, procédé qui comprend les étapes consistant :

    i) à engendrer un spectre quantique double de protons bidimensionnel ou un spectre quantique double pondéré de relaxation DQC (cohérence quantique double) d'une solution comprenant une ou plusieurs molécules chimiques contenant au moins deux doublets ;
    ii) à engendrer un spectre quantique double de protons bidimensionnel ou un spectre quantique double pondéré de relaxation DQC de la solution précitée comprenant en outre la biomolécule cible ;
    iii) à comparer le spectre en (i) avec le spectre en (ii) de manière à détecter toutes différences qui identifient la présence d'une ou plusieurs molécules chimiques qui se lient à la biomolécule cible, le spectre quantique double de protons bidimensionnel étant ou non refocalisé.

2.  Procédé suivant la revendication 1, comprenant l'étape supplémentaire consistant à engendrer deux spectres quantiques doubles de protons bidimensionnels avec et sans la protéine cible pour le composé, ou pour chacun des composés, qui ont été identifiés comme ligands à la biomolécule cible.

3.  Procédé suivant la revendication 2, dans lequel les différences qui identifient la présence d'une ou plusieurs molécules chimiques qui se lient à la biomolécule cible sont des différences dans le déplacement chimique ou l'intensité d'un ou plusieurs signaux.

4.  Procédé suivant l'une quelconque des revendications précédentes, dans lequel chacun des deux spectres quantiques doubles de protons bidimensionnels ou spectres quantiques doubles pondérés de relaxation DQC est enregistré avec des gradients de champ pulsé à angle magique ou z.

5.  Procédé suivant l'une quelconque des revendications précédentes, dans lequel les spectres quantiques doubles de protons bidimensionnels ou spectres quantiques doubles pondérés de relaxation DQC sont enregistrés dans des solvants non deutérés.

6.  Procédé suivant la revendication 1, dans lequel chaque composé d'essai est présent dans l'échantillon en un rapport molaire à la biomolécule cible d'environ 1:1 à environ 20:1.

7.  Procédé suivant la revendication 1, dans lequel la biomolécule cible est libre en solution, dissoute dans des détergents ou des micelles, ou couplée à une résine.

8.  Procédé suivant la revendication 1, dans lequel la biomolécule cible est une protéine, un anticorps ou un fragment d'ADN ou d'ARN.

9.  Procédé suivant la revendication 8, dans lequel la biomolécule cible est une protéine.

10. Procédé suivant la revendication 9, dans lequel la protéine est le domaine SH2 de la protéine Grb2.

Fig. 1

EP 1 224 486 B1

Fig. 2

EP 1 224 486 B1

**Fig. 3**

Fig. 4

Fig. 5

EP 1 224 486 B1